# EUROPÄISCHE PATENTSCHRIFT

(11) **EP 3 237 010 B1**
(45) Veröffentlichungstag und Bekanntmachung des Hinweises auf die Patenterteilung: **14.08.2019**
(21) Anmeldenummer: 16718843.2
(22) Anmeldetag: 07.04.2016
(51) Int. Cl.: A61K 41/00, A61K 47/58, A61K 47/60, A61P 35/00, A61K 9/00, A61K 9/08, A61K 9/19, A61K 47/32

(54) **FORMULIERUNG VON HYPERICIN ZUR PHOTODYNAMISCHEN THERAPIE**
FORMULATION OF HYPERICIN FOR PHOTODYNAMIC THERAPY
FORMULATION D'HYPÉRICINE POUR LA THÉRAPIE PHOTODYNAMIQUE

(30) Priorität: 28.09.2015 AT 6302015
(43) Veröffentlichungstag der Anmeldung: 01.11.2017
(73) Patentinhaber: Sanochemia Pharmazeutika AG, 1090 Wien (AT)
(72) Erfinder: ABRAHAMSBERG, Christina, 1030 Wien (AT); FRANTSITS, Werner, 1130 Wien (AT); GERDES, Klaus, 41564 Kaarst Vorst (DE); GUNGL, Jòzsef, 9423 Àgfalva (HU); KÄLZ, Beate, 7035 Steinbrunn (AT); MEDINGER, Gregor, Sag Harbor, NY 11963 (US); WELZIG, Stefan, 1030 Wien (AT)
(74) Vertreter: Beer & Partner Patentanwälte KG
(86) Internationale Anmeldenummer: PCT/AT2016/000033
(87) Internationale Veröffentlichungsnummer: WO 2017/054017

(56) Entgegenhaltungen:
- WO-A1-2009/066294
- WO-A1-2015/131891
- WO-A2-01/89576
- HUYGENS A ET AL: "Stability of different formulations and ion pairs of hypericin", EUROPEAN JOURNAL OF PHARMACEUTICS AND BIOPHARMACEUTICS, ELSEVIER SCIENCE PUBLISHERS B.V., AMSTERDAM, NL, Bd. 59, Nr. 3, 1. April 2005 (2005-04-01), Seiten 461-468, XP027805033, ISSN: 0939-6411 [gefunden am 2005-04-01]
- ANDREAS KUBIN ET AL: "Fluorescence Diagnosis of Bladder Cancer with New Water Soluble Hypericin Bound to Polyvinylpyrrolidone: PVP-Hypericin", PHOTOCHEMISTRY AND PHOTOBIOLOGY, Bd. 84, Nr. 6, 1. November 2008 (2008-11-01), Seiten 1560-1563, XP055285844, US ISSN: 0031-8655, DOI: 10.1111/j.1751-1097.2008.00384.x
- DANIELA FEINWEBER ET AL: "Applicability of new degradable hypericin-polymer-conjugates as photosensitizers: principal mode of action demonstrated by in vitro models", PHOTOCHEMICAL AND PHOTOBIOLOGICAL SCIENCES, Bd. 13, Nr. 11, 1. Januar 2014 (2014-01-01), Seiten 1607-1620, XP055286452, GB ISSN: 1474-905X, DOI: 10.1039/C4PP00251B
- JOACHIM VANDEPITTE ET AL: "Biodistribution of PVP-hypericin and hexaminolevulinate-induced PpIX in normal and orthotopic tumor-bearing rat urinary bladder", CANCER CHEMOTHERAPY AND PHARMACOLOGY., Bd. 67, Nr. 4, 1. April 2011 (2011-04-01), Seiten 775-781, XP055286423, BERLIN. ISSN: 0344-5704, DOI: 10.1007/s00280-010-1375-0 in der Anmeldung erwähnt

## Beschreibung

Die Erfindung betrifft eine Formulierung zum Herstellen einer Instillationslösung zur Verwendung bei der photodynamischen Therapie, enthaltend an einen polymeren Komplexbildner, nämlich ein Polyethylenglykol oder ein Poly-N-Vinylamid gebundenes Hypericin in Form eines Alkalimetallsalzes, insbesondere in Form des Kaliumsalzes oder des Natriumsalzes, gemäß den Ansprüchen.

Die photodynamische Therapie (PDT) ist ein Verfahren, das zur Behandlung von Tumoren und prämalignen Veränderungen der Haut und Mukosa verschiedener Hohlorgane geeignet ist (Juarranz et al., 2008; Agostinis et al., 2011).

Die PDT beruht auf der Wechselwirkung dreier Komponenten: Photosensibilisator, Licht im sichtbaren Bereich und Sauerstoff.

Nach systemischer oder topischer Applikation eines Photosensibilisators erfolgt eine Akkumulation des Photosensibilisators im malignen Gewebe. Mit Hilfe von Licht geeigneter Wellenlänge kann der Photosensibilisator angeregt werden. Im angeregten Zustand wird Energie auf einen Reaktionspartner, z.B. molekularen Sauerstoff, übertragen. Dabei werden reaktive Sauerstoffmoleküle generiert, die wiederum zelluläre Strukturen des Tumorgewebes schädigen, wodurch zelluläre Prozesse wie Apoptose und Nekrose eingeleitet werden (Agostinis, et al., 2011; Allison and Sibata, 2010).

Ein idealer Photosensibilisator für die PDT zeigt selektive Akkumulation in Tumorzellen, keine oder minimale systemische Toxizität und ist photochemisch effizient.

Hypericin 1,3,4,6,8,13-Hexahydroxy-10,11-dimethylphenanthro (1,10,9,8-opqra)perylene-7,14-dion wurde in der Literatur bereits als potentieller Photosensibilisator beschrieben (Agostinis et al., 2002).

In *in vitro* Untersuchungen konnte die Wirksamkeit von Hypericin in der PDT in einer Reihe von Zelllinien gezeigt werden (Karioti und Bilia, 2010).

Darüber hinaus bestätigen *in vivo* Tierstudien das Potential Hypericins für eine Anwendung in der PDT (Bhuvaneswari et al., 2010; Chen et al., 2003; Liu et al., 2000; Sanovic et al., 2011).

Hypericin ist hydrophob und in Wasser unlöslich. Aus diesem Grund wurde in der Vergangenheit Hypericin mit Hilfe des organischen Lösungsmittels Dimethylsulfoxid (DMSO) oder eines wasserlöslichen Polymers, Polyethylenglycol (PEG), in Lösung gebracht.

Tierexperimente in einem Rattenmodell zeigten ermutigende Ergebnisse hinsichtlich der PDT des Blasenkarzinoms. Dabei wurde Hypericin mit Hilfe von Polyethylenglykol in die Tumorzellen gebracht. Mit einer Hypericindosis von 30 µM und einer Bestrahlung mit Licht (595 nm) einer Intensität von 25 bis zu 50 mW/cm² konnten bis zu 98% der Tumorzellen abgetötet werden (Kamuhabwa et al. 2003).

Für eine klinische Anwendung wird jedoch eine wasserlösliche Formulierung von Hypericin benötigt, die Tumorselektivität besitzt, und mit Licht im sichtbaren Bereich angeregt werden kann.

Das Dokument WO 01/89576 A2 beschreibt, wie die Löslichkeit von Hypericin durch den Hilfsstoff Polyvinylpyrrolidon (Povidon, PVP) erhöht werden kann.

Die Verwendung von PVP-Hypericin in der PDT ist auch in der WO 2014/079972 A1 beschrieben. Die WO 2014/079972 A1 befasst sich insbesondere mit einem in der PDT von Hohlorganen, wie der menschlichen Blase, einsetzbarem Gerät.

Die WO 2015/131891 A1 betrifft die Verwendung von Hypericin in Form des Natriumsalzes sowohl für die photodynamische Diagnose als auch für die photodynamische Therapie.

Das in WO 2015/131891 A1 geoffenbarte Produkt liegt in Form einer wasserfreien Lösung in Alkohol (nämlich Ethanol) vor und enthält als Lösungsvermittler PEG. Die Lösung ist vor ihrer Verwendung als Photosensitizer in einer Trägerlösung (Glukoselösung) zu verdünnen.

Die so erhaltene Infusionslösung soll 0,01 bis 0,05 mg Natrium-Hypericinat enthalten und soll über einen Zeitraum von 90 Minuten administriert werden.

PVP-Hypericin zeigt eine selektive Akkumulation in Tumorzellen *in vitro* und *in vivo* (Kubin et al., 2008; Vandepitte et al., 2011).

Der Erfindung liegt die Aufgabe zu Grunde, eine sterile und stabile Formulierung von Hypericin, die zur klinischen Anwendung in der PDT einsetzbar ist, zur Verfügung zu stellen.

Gelöst wird diese Aufgabe mit einer Formulierung von Hypericin, welche die Merkmale von Patentanspruch 1 aufweist.

Bevorzugte und vorteilhafte Ausführungsformen der erfindungsgemäßen Formulierung von Hypericin sind Gegenstand der Unteransprüche.

Überraschenderweise hat sich gezeigt, dass die erfindungsgemäße Formulierung von Hypericin nur dann stabil und damit unter klinischen Bedingungen anwendbar ist, wenn Hypericin als Salz vorliegt.

Eine Evaluierung der erfindungsgemäßen Formulierung von Hypericin in Tierexperimenten hat überraschenderweise gezeigt, dass bei einer Dosis von 30 µM Hypericin in einer stabilen Formulierung mit PVP gemäß Beispiel 1 eine benötigte Lichtintensität von 5 oder 25 mW/cm² bei einer Wellenlänge von 595 nm und 120 Minuten Einwirkzeit in der Blase (Instillationszeit) ausreicht, um 98% der Tumorzellen abzutöten. Das gleiche Ergebnis von 98% abgetöteten Tumorzellen wird auch bei gleicher Lichtintensität und 40 µM Hypericin bei 15 oder 30 Minuten Einwirkzeit und Behandlung mit Licht einer Wellenlänge von 610 nm erzielt. Ebenso erzielt eine Instillationszeit von 1 Stunde, bei 20 µM Hypericin, gleicher Lichtintensität und 570 nm Lichtfrequenz eine Abtötungsrate von 97% und eine Instillationszeit von 120 Minuten, bei 9 µM Hypericin, gleicher Lichtintensität und Behandlung bei 600 nm eine Abtötungsrate von 95%. Womit bei Lichtintensitäten von 5 bis 25 mW/cm² bei Lichtfrequenzen von 570 bis 610 nm, Hypericinkonzentrationen von 9 bis 40 µM, Einwirkzeiten zwischen 15 und 120 Minuten eine Abtötungsrate von 95 bis 98% der Tumorzellen (Anwendungsbesipiele 1, 2, 3 und 4) erzielt wird.

Die Effektivität einer PDT ist wesentlich von der Gesamtlichtmenge abhängig. Gleichzeitig ist mit steigender Lichtintensität die Wahrscheinlichkeit von lokalen Nebenwirkungen erhöht.

Mit Hilfe der erfindungsgemäßen Formulierung wird eine verbesserte Akkumulation im malignen Gewebe erreicht, wodurch eine deutlich verminderte Lichtintensität von bereits 5 bis maximal 25 mW/cm² ausreicht, um die Tumorzellen abzutöten.

Die selektive Anreicherung der erfindungsgemäßen Formulierung von Hypericin und die überraschenderweise niedrige Lichtintensität, die für eine PDT im Tiermodell beim Anwenden der erfindungsgemäßen Formulierung von Hypericin benötigt wurde, erlaubt die Anwendung in der Therapie von Läsionen in verschiedenen Kävitäten des Körpers, die mit der erforderlichen Lichtdosis erreicht werden können.

Nachstehend werden Beispiele der erfindungsgemäßen Formulierung von Hypericin (Hypericin-PVP-Komplex) wiedergegeben.

Allgemeine Verfahrensweise zum Herstellen einer Formulierung mit dem Wirkstoff Natrium-Hypericinat:
Ziel ist das Herstellen einer Hypericin enthaltenden Formulierung zur Anwendung als Photosensibilisator im Bereich der photodynamischen Therapie.

Die erfindungsgemäße Formulierung wird aus einem Salz von Hypericin, insbesondere aus Natrium-Hypericinat, hergestellt.

Um den Hypericin-Gehalt des Ausgangsmaterials zu definieren, werden neben der Gehaltsbestimmung vor allem Wassergehalt und im Falle von Natrium-Hypericinat der Natrium Anteil festgehalten. Die chemisch-physikalischen Eigenschaften können einen Einfluss auf die Formulierung des pharmazeutischen Arzneimittels haben.

Für die klinische Anwendung ist eine Stabilität der erfindungsgemäßen Formulierung erforderlich. Die Stabilität wird durch die Zusammensetzung des Fertigproduktes gewährleistet und betrifft gleichzeitig auch das Herstellungsverfahren. Durch die verwendeten Puffersysteme kann auch während des Herstellens bis zum Lyophilisieren des Fertigproduktes eine hinreichende Stabilität der Bulklösung erreicht werden.

Als Puffersysteme können verschiedene Zusatzstoffe herangezogen werden, die bevorzugt sowohl für die Bulklösung als auch für die rekonstituierte Lösung einen physiologisch verträglichen pH-Wert und einen osmotischen Druck nach der Rekonstitution mit 50 ml Aqua ad Injectabilia von 290 mOsmol/kg erzielen. Phosphat- oder Zitrat-Puffersysteme können in erster Linie zum Einsatz kommen.

Nach dem Fertigstellen der Bulklösung aus den oben genannten Bestandteilen wird die entsprechende Menge der Bulklösung in Injektionsflaschen abgefüllt und lyophilisiert.

### Beispiel 1:

Aus dem Natrium-Hypericinat wird eine Lösung mit einer Zieleinwaage von 90,0 mg Hypericin hergestellt.

Zu 1875 mg PVP k25 werden 5,0 g der Hypericin-Lösung zugesetzt und vollständig gelöst.

Diese Lösung wird quantitativ mit einer Phosphatpufferlösung auf 250,0 g aufgefüllt. Die Endkonzentration dieser Lösung ist 0,0225 mg Hypericin/g Lösung.

Für das Lyophilisieren wird eine definierte Menge der so erhaltenen Bulklösung in Injektionsflaschen abgefüllt und mit einem entsprechenden Lyo-Programm das fertige Lyophilisat hergestellt.

Beispiel 2:

Es wird so wie im Beispiel 1 angegeben gearbeitet, wobei statt PVP k25 zum Komplexieren vom Natrium-Hypericinat PVP k17 verwendet wird.

### Beispiel 3:

Es wird so wie im Beispiel 1 angegeben gearbeitet, wobei statt PVP k25 zum Komplexieren vom Natrium-Hypericinat PVP k30 verwendet wird.

### Beispiel 4:

Es wird so wie in Beispiel 1, 2 oder 3 angegeben gearbeitet, wobei statt der Phosphatpufferlösung eine Zitronensäure-Pufferlösung verwendet wird.

Die wie in den Beispielen 1 bis 4 beschrieben hergestellten Bulklösungen können mit unterschiedlichen Hypericin-Gehalten produziert werden.

Die Wirksamkeit der erfindungsgemäßen Formulierung von Hypericin wurde in einer präklinischen Studie unter Verwenden der Formulierung als Beispiel 1 überprüft.

### Anwendungsbeispiele:

Dazu wurde die erfindungsgemäße Formulierung von Hypericin für die PDT in einem präklinischen, orthotopen Blasentumormodell in Ratten untersucht. Bei allen Beispielen wurden die Tumore mit der erfindungsgemäßen Formulierung von Hypericin in unterschiedlichen Konzentrationen von 9 bis 40 µM
, bei unterschiedlichen Lichtintensitäten von 5 oder 25 mW/cm², unterschiedlichen Lichtfrequenzen von 570 bis 610 nm und unterschiedlichen Instillationszeiten behandelt.

Beispiel 1. Nach einer 2-stündigen Instillation mit 30 µM der erfindungsgemäßen Formulierung von Hypericin und unterschiedlichen Lichtintensitäten (5 oder 25 mW/cm²) mit Licht einer Wellenlänge von 595 nm konnten bis zu 98% der Tumorzellen abgetötet werden.

Beispiel 2. Nach einer 1-stündigen Instillation mit 20 µM der erfindungsgemäßen Formulierung von Hypericin und unterschiedlichen Lichtintensitäten (5 oder 25 mW/cm²) mit Licht der Wellenlänge 570 nm konnten bis zu 97% der Tumorzellen abgetötet werden.

Beispiel 3. Nach einer 15 oder 30 Minuten Instillation mit 40 µM der erfindungsgemäßen Formulierung von Hypericin und unterschiedlichen Lichtintensitäten (5 oder 25 mW/cm²) mit Licht der Wellenlänge von 610 nm, konnten bis zu 98% der Tumorzellen abgetötet werden.

Beispiel 4. Nach einer 2-stündigen Instillation mit 9 µM der erfindungsgemäßen Formulierung von Hypericin und unterschiedlichen Lichtintensitäten (5-25 mW/cm²) mit Licht der Wellenlänge 600 nm konnten bis zu 95% der Tumorzellen abgetötet werden.

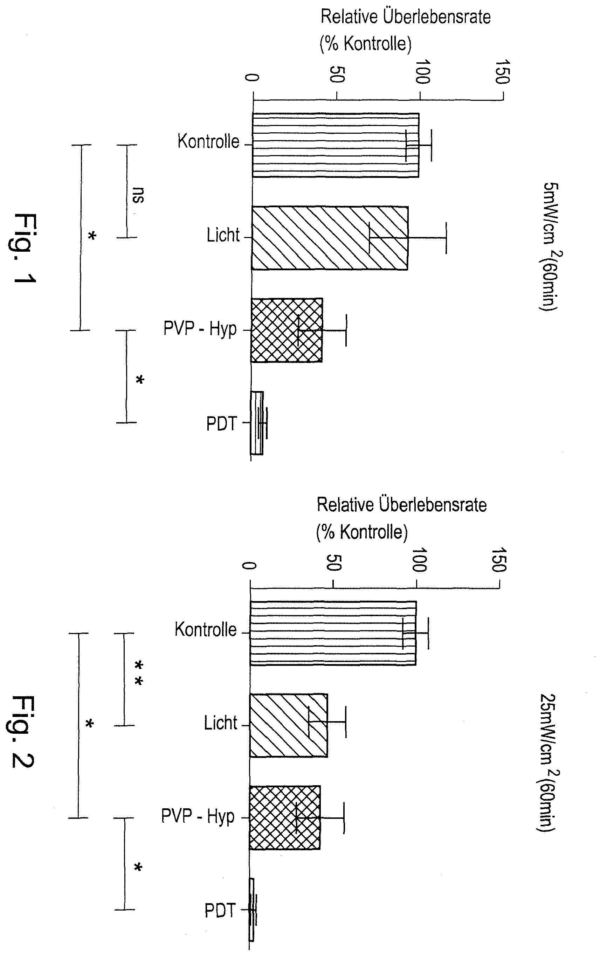

Die Ergebnisse der Untersuchungen am Rattenmodell sind in den Fig. 1 und 2 wiedergegeben. In den Diagrammen steht "ns" für "nicht signifikant" und "*" für "signifikant". Die Diagramme der Fig. 1 und 2 zeigen das Überleben von Tumorzellen nach Behandeln mit der erfindungsgemäßen Formulierung von Hypericin und Licht. 24 Stunden nach der Behandlung wurde das Blasengewebe dissoziiert und die überlebenden Zellen mit Hilfe eines klonogenen Assays im Vergleich zur Kontrolle (ohne PVP-Hypericin und Licht) bestimmt.

Das relative Überleben der Zellen unter PDT Bedingungen (PVP-Hypericin gemäß Beispiel 1 und Behandlung mit Licht) beträgt (dargestellt als Mittelwert + SD): 7.4 (+/- 6.4) % bei Verwendung von 5 mW/cm² und 2.4 (+/- 4.0) % bei 25 mW/cm² und einer Behandlungsdauer mit Licht von 60 Minuten. Dies ist in zwei Diagrammen (Fig. 1 und 2) dargestellt.

### Referenzen:

Agostinis P, Berg K, Cengel KA, Foster TH, Girotti AW, Gollnick SO, Hahn SM, Hamblin MR, Juzeniene A, Kessel D, Korbelik M, Moan J, Mroz P, Nowis D, Piette J, Wilson BC, Golab J. Photodynamic therapy of cancer: an update. CA Cancer J Clin. 2011 Jul-Aug; 61 (4):250-281
Agostinis P, Vantieghem A, Merlevede W, de Witte PA. Hypericin in cancer treatment: more light on the way. Int J Biochem Cell Biol. 2002 Mar;34(3):221-241
Allison RR, Sibata CH. Oncologic photodynamic therapy photosensitizers: a clinical review. Photodiagnosis Photodyn Ther. 2010 Jun;7(2):61-75
Bhuvaneswari R, Thong PS, Gan YY, Soo KC, Olivo M. Evaluation of hypericin-mediated photodynamic therapy in combination with angiogenesis inhibitor bevacizumab using in vivo fluorescence confocal endomicroscopy. J Biomed Opt. 2010 Jan-Feb;15(1):011114. Erratum in: J Biomed Opt. 2010
Chen B, Ahmed B, Landuyt W, Ni Y, Gaspar R, Roskams T, de Witte PA. Potentiation of photodynamic therapy with hypericin by mitomycin C in the radiation-induced fibrosarcoma-1 mouse tumor model. Photochem Photobiol. 2003 Sep; 78 (3) :278-282.
Juarranz A, Jaen P, Sanz-Rodriguez F, Cuevas J, González S. Photodynamic therapy of cancer. Basic principles and applications. Clin Transl Oncol. 2008 Mar; 10 (3) :148-154
Karioti A, Bilia AR. Hypericins as potential leads for new therapeutics. Int J Mol Sci. 2010 Feb 4;11(2):562-594
Kubin A, Meissner P, Wierrani F, Burner U, Bodenteich A, Pytel A, Schmeller N. Fluorescence diagnosis of bladder cancer with new water soluble hypericin bound to polyvinylpyrrolidone: PVP-hypericin. Photochem Photobiol. 2008; 84(6):1560-1563
Kamuhabwa AA, Roskams T, D'Hallewin MA, Baert L, Van Poppel H, de Witte PA. Whole bladder wall photodynamic therapy of transitional cell carcinoma rat bladder tumors using intravesically administered hypericin. Int J Cancer. 2003 Nov 10; 107 (3) :460-467
Liu CD, Kwan D, Saxton RE, McFadden DW. Hypericin and photodynamic therapy decreases human pancreatic cancer in vitro and in vivo. J Surg Res. 2000 Sep;93(1):137-143
Sanovic R1, Verwanger T, Hartl A, Krammer B. Low dose hypericin-PDT induces complete tumor regression in BALB/c mice bearing CT26 colon carcinoma. Photodiagnosis Photodyn Ther. 2011 Dec; 8 (4) :291-296
Vandepitte J, Van Cleynenbreugel B, Hettinger K, Van Poppel H, de Witte PA. Biodistribution of PVP-hypericin and hexaminolevulinate-induced PpIX in normal and orthotopic tumorbearing rat urinary bladder. Cancer Chemother Pharmacol. Cancer Chemother Pharmacol. 2011 Apr;67(4):775-781
Vandepitte J, Roelants M, Van Cleynenbreugel B, Hettinger K, Lerut E, Van Poppel H, de Witte PA. Biodistribution and photodynamic effects of polyvinylpyrrolidone-hypericin using multicellular spheroids composed of normal human urothelial and T24 transitional cell carcinoma cells. J. Biomed Opt. 2011 Jan-Feb; 16 (1) :018001

## Patentansprüche

1. Formulierung zum Herstellen einer Instillationslösung, die bei der photodynamischen Therapie verwendbar ist, enthaltend an einen polymeren Komplexbildner, nämlich ein Polyethylenglykol oder ein Poly-N-Vinylamid gebundenes Hypericin in Form eines Alkalimetallsalzes, insbesondere in Form des Kaliumsalzes oder des Natriumsalzes, wobei die Instillationslösung Hypericin in einer Konzentration von 9 bis 40 µM enthält, **dadurch gekennzeichnet, dass** die Formulierung ein stabiles Lyophilisat ist, das aus einer wässerigen Lösung, enthaltend das Alkalimetallsalz von Hypericin, den Komplexbildner und ein Puffersystem, insbesondere einen Phosphatpuffer oder eine Zitronensäure-Pufferlösung, gewonnen ist.

2. Formulierung nach Anspruch 1, **dadurch gekennzeichnet, dass** das Poly-N-vinylamid Polyvinylpyrrolidon (PVP)verschiedenen Polymerisations- und Vernetzungsgrades ist.

3. Verfahren zum Herstellen einer stabilen Formulierung nach Anspruch 1 oder 2, **gekennzeichnet durch** nachstehende, aufeinanderfolgende Verfahrensschritte,
a) es wird eine wässerige Lösung des Alkalimetallsalzes von Hypericin hergestellt,
b) die in Schritt a) erhaltene Lösung wird zu dem Komplexbildner, insbesondere Polyvinylpyrrolidon zugegeben und gelöst,
c) zu der in Schritt b) erhaltenen Lösung wird ein Phosphatpuffer oder eine Zitronensäure-Pufferlösung zugesetzt, um eine Konzentration von 0,0225 mg Hypericin/g Lösung zu erhalten und
d) Anteile der nach Schritt c) erhaltenen Bulk-Lösung werden lyophilisiert.

## Claims

1. Formulation for preparing an instillation solution useful in photodynamic therapy, containing hypericin bound to a polymeric complexing agent, namely a polyethylene glycol or a poly-N-vinylamide, in the form of an alkali metal salt, in particular in the form of the potassium salt or the sodium salt, wherein the instillation solution contains hypericin in a concentration of 9 to 40 µM, **characterized in that** the formulation is a stable lyophilisate obtained from an aqueous solution containing the alkali metal salt of hypericin, the complexing agent and a buffer system, in particular a phosphate buffer or a citric acid buffer solution.

2. Formulation according to claim 1, **characterized in that** the poly-N-vinylamide is polyvinylpyrrolidone (PVP) of different degrees of polymerization and crosslinking.

3. Method for preparing a stable formulation according to claim 1 or 2, **characterized by** the following successive method steps,
a) an aqueous solution of the alkali metal salt of hypericin is prepared,
b) the solution obtained in step a) is added to the complexing agent, in particular polyvinylpyrrolidone, and dissolved,
c) a phosphate buffer or a citric acid buffer solution is added to the solution obtained in step b) to obtain a concentration of 0.0225 mg hypericin/g solution, and
d) fractions of the bulk solution obtained in step (c) are lyophilized.

## Revendications

1. Formule pour la fabrication d'une solution pour instillation utilisable pour une thérapie photodynamique, contenant une hypéricine sous la forme d'un sel de métal alcalin, en particulier sous forme de sel potassique ou de sel sodique, liée un agent complexant polymère, à savoir du polyéthylène-glycol ou du poly-N-vinylamide, dans laquelle la solution pour instillation contient de l'hypéricine à une concentration de 9 à 40 µM, **caractérisée en ce que** la formule est un lyophilisat stable obtenu à partir d'une solution aqueuse contenant le sel de métal alcalin d'hypéricine, l'agent complexant et un système de tampon, en particulier un tampon phosphate ou une solution tamponnée d'acide citrique.

2. Formule selon la revendication 1, **caractérisée en ce que** le poly-N-vinylamide est de la polyvinylpyrrolidone (PVP) à différents degrés de polymérisation et de réticulation.

3. Procédé pour la fabrication d'une formule stable selon la revendication 1 ou 2, **caractérisé en ce qu'**il comprend les étapes de procédé successives suivantes :
a) une solution aqueuse du sel de métal alcalin d'hypéricine est fabriquée,
b) la solution obtenue dans l'étape a) est ajoutée à l'agent complexant, en particulier la polyvinylpyrrolidone et dissoute,
c) à la solution obtenue dans l'étape b) est ajouté un tampon phosphate ou une solution tamponnée d'acide citrique pour obtenir une concentration de 0,0225 mg d'hypéricine/g de solution et
d) portions de la solution mère obtenue dans l'étape c) sont lyophilisés.
